# EUROPÄISCHE PATENTSCHRIFT

(11) **EP 1 736 179 B1**
(45) Veröffentlichungstag und Bekanntmachung des Hinweises auf die Patenterteilung: **02.11.2011**
(21) Anmeldenummer: 06011580.5
(22) Anmeldetag: 03.06.2006
(51) Int. Cl.: A61L 24/06, A61L 27/16

(54) **Polymethylmethacrylat-Knochenzement**
Polymethylmethacrylate bone cement
Ciment osseux à base d'un polymère de méthacrylate de méthyle

(30) Priorität: 22.06.2005 DE 102005029218; 13.07.2005 DE 102005033210
(43) Veröffentlichungstag der Anmeldung: 27.12.2006
(73) Patentinhaber: Heraeus Kulzer GmbH, 63450 Hanau (DE)
(72) Erfinder: Kühn, Klaus-Dieter, 35041 Marburg (DE); Vogt, Sebastian, 99092 Erfurt (DE)
(74) Vertreter: Kühn, Hans-Christian

(56) Entgegenhaltungen:
- US-A- 4 588 583
- LEE C L ET AL: "LASER ABLATION OF DYED ACRYLIC BONE CEMENT" LASERS IN SURGERY AND MEDICINE, WILEY- LISS, NEW YORK, US, Bd. 20, Nr. 3, 1. Januar 1997 (1997-01-01), Seiten 280-289, XP000694435 ISSN: 0196-8092
- BARGAR W L; MARTIN R B; DEJESUS R; MADISON M T: "The addition of tobramycin to contrast bone cement. Effect on flexural strength." THE JOURNAL OF ARTHROPLASTY, Bd. 1, Nr. 3, 1986, Seiten 165-168, XP002557137 USA

## Beschreibung

Der Gegenstand der Erfindung ist ein Polymethylmethacrylat-Knochenzement.

Polymethylmethacrylat-Knochenzemente (PMMA-Knochenzemente) finden seit Jahrzehnten breite Anwendung in der Medizin zur Verankerung von Endoprothesen im Knochen (Klaus-Dieter Kühn: Knochenzemente für die Endoprothetik: ein aktueller Vergleich der physikalischen und chemischen Eigenschaften handelsüblicher PMMA-Zemente. Springer Verlag Berlin Heidelberg New York, 2001). Polymethylmethacrylat-Knochenzemente sind im Allgemeinen aus einer flüssigen Monomerkomponente und einer Pulverkomponente aufgebaut. Die flüssige Monomerkomponente besteht aus Methylmethacrylat und einem Aktivator. Als Aktivator wird N,N-Dimethyl-p-toluidin bevorzugt verwendet. Die Pulverkomponente besteht aus Polymethylmethacrylat oder Polymethyl-co-acrylmethacrylat, einem Röntgenkontrastmittel und einem radikalischen Initiator. Als Röntgenkontrastmittel sind Zirkoniumdioxid und Bariumsulfat üblich. Dibenzoylperoxid wird als radikalischer Initiator bevorzugt eingesetzt. Nach Vermischen der Monomerkomponente und der Pulverkomponente härtet der Knochenzement durch radikalische Polymerisation des Monomers innerhalb weniger Minuten aus.

Übliche Polymethylmethacrylat-Knochenzemente liegen nach dem Anmischen als weiße bis schwach gelbliche pastöse Massen vor. Dadurch ist mitunter eine optische Differenzierung zwischen dem Knochenzement und dem Knochengewebe problematisch. Es ist jedoch wünschenswert, dass der Knochenzement visuell problemlos vom umliegenden Knochengewebe unterschieden werden kann. Aus diesem Grund werden die PMMA-Knochenzemente der Firma Heraeus Kulzer seit ungefähr 30 Jahren mit Chlorophyll grün gefärbt. Chlorophyll ist jedoch in dem verwendeten Monomer Methylmethacrylat in nur sehr geringem Umfang löslich. Deshalb wird zur Verbesserung der Löslichkeit raffiniertes Erdnussöl zugesetzt. Das raffinierte Erdnussöl ist proteinfrei. Problematisch sind jedoch, wie bei jedem komplexen Naturprodukt, Schwankungen in der Zusammensetzung. Bisher wurden noch keine Alternativen zum raffinierten Erdnussöl gefunden.

Wichtig bei der Färbung von PMMA-Knochenzementen ist neben der toxikologischen Unbedenklichkeit des Farbstoffs die homogene Verteilung des Farbstoffs im gesamten Zement. Aus diesem Grund haben bisher Knochenzemente, die in der Monomerflüssigkeit oder im der Pulverkomponente Farbstoffpigmente enthalten, keine breite Anwendung gefunden. Farbstoffpigmente sind mit der prinzipiellen Gefahr verbunden, dass sich die Pigmentpartikel aus der Zementmatrix herauslösen und migrieren können. Es ist deshalb besonders wichtig, dass der Farbstoff homogen im Knochenzement verteilt und fest eingeschlossen ist.

Der Erfindung liegt deshalb die Aufgabe zu Grunde, einen gefärbten PMMA-Knochenzement zu entwickeln. Die flüssige Monomerkomponente des PMMA-Knochenzements soll keine komplexen Naturprodukte als Löslichkeitsvermittler für in Methylmethacrylat nicht oder nur gering lösliche Farbstoffe enthalten. Die flüssige Monomerkomponente soll einen homogen verteilten, sichtklar gelösten Farbstoff enthalten.

In dem Artikel von C. Lee et al., Lasers in Surgery and Medicine 20, 280-289 (1997), wird ein Polymethylmethacrylat-Knochenzement mit einem blauen oder roten Farbstoff in der Pulverkomponente beschrieben.

Die Aufgabe der Erfindung wird durch einen Knochenzement gemäß Anspruch 1 gelöst. Bevorzugt sind in der Monomerflüssigkeit 0,001-1,00 Masseprozent eines in Methacrylsäuremethylester nicht oder gering löslichen Farbstoffs oder Farbstoffgemisches und 0,001-2,00 Masseprozent eines synthetisch hergestellten, proteinfreien, hydrophoben, niedermolekularen oder oligomeren Lösungsvermittlers für den Farbstoff oder das Farbstoffgemisch gelöst.
Die Monomerflüssigkeit ist bei Raumtemperatur sichtklar.
Vorzugsweise sind 0,001-1,00 Masseprozent eines in Methacrylsäuremethylester nicht oder gering löslichen Farbstoffs oder Farbstoffgemisches und 0,001-2,00 Masseprozent eines synthetisch hergestellten, proteinfreien, hydrophoben, niedermolekularen oder oligomeren Lösungsvermittlers, der bei Raumtemperatur flüssig oder pastös ist, homogen im Polymethacrylat oder Polymethylacrylat der Pulverkomponente gelöst.
Zur Erfindung gehört dabei auch die Verwendung von Copolymeren, die durch Polymerisation von Acrylsäureestern und/oder Methacrylsäureestern und Styren oder Styrenderivaten hergestellt wurden.
Erfindungsgemäße Knochenzemente können zusätzlich Wirkstoffe wie Antibiotika enthalten - oder aber von solchen Wirkstoffen frei sein.

Es können auch zwei synthetisch hergestellte, proteinfreie, hydrophobe, niedermolekulare oder oligomere Lösungsvermittler eingesetzt werden, um einen Farbstoff in der Monomerflüssigkeit lösen zu können.

Es ist zweckmäßig, dass der Farbstoff/Farbstoffgemisch und der synthetisch hergestellte, proteinfreie, hydrophobe, niedermolekulare oder oligomere Lösungsvermittler mit dem Farbstoff/Farbstoffgemisch in der Monomerflüssigkeit lösliche Addukte bildet. Im Rahmen der Erfindung ist dabei besonders die Adduktebildung auf Grund von unpolaren Wechselwirkungen und die Bildung von Addukten durch Addition von Aminogruppen oder Thiolgruppen an Doppelbindungen im Sinne einer Michael-Addition. Ebenfalls im Sinne der Erfindung ist die Ausbildung von Addukten durch kovalente Verknüpfung durch Bildung von Carbonsäureamiden, Carbonsäureestern, Ethern, Azomethinen und Chelaten.

Außerdem ist zweckmäßig, dass Ölsäureester und/oder Elaidinsäureester und/oder Linolsäureester und/oder Linolensäureester der aliphatischen Alkohole mit 1 bis 22 Kohlenstoffatomen oder Oligomere dieser Ölsäureester als Löslichkeitsvermittler bevorzugt sind.

Zweckmäßig ist, dass Methacrylsäureester oder Acrylsäureester der aliphatischen Alkohole mit 4 bis 16 Kohlenstoffatomen oder Oligomere dieser Methacrylsäureester mit einer Molmasse kleiner 3000 g/mol als Löslichkeitsvermittler bevorzugt sind. Im Sinne der Erfindung ist ebenfalls die Verwendung von Maleinsäureestern, Fumarsäureestern, Itaconsäureestern und Sorbinsäureestern als Löslichkeitsvermittler.

Weiterhin ist zweckmäßig, dass Glycerintrioleat, Glycerintrilinolat, Glycerintrilinolenat und Glycerintrielaidinat als Löslichkeitsvermittler bevorzugt sind. Im Rahmen der Erfindung ist auch die Verwendung von Glycerinmischestern dieser ungesättigten Fettsäuren.

Es ist außerdem zweckmäßig, dass der Löslichkeitsvermittler radikalisch polymerisierbare Doppelbindungen enthält. Es können ein oder mehrere Doppelbindungen im Löslichkeitsvermittler enthalten sein. Der besondere Vorteil ist darin zu sehen, dass der Löslichkeitsvermittler während der Aushärtung des PMMA-Knochenzementes an der radikalischen Polymerisation des Methylmethacrylates mit teilnimmt und dadurch in die entstehenden Polymerketten mit eingebaut wird. Dadurch ist eine Migration des Löslichkeitsvermittlers aus dem ausgehärteten PMMA-Knochenzement nicht möglich.

Als Farbstoffe sind Chlorophyll, Indigo, Malachitgrün, Kristallviolett, Kupferphthalocyanin, Kobaltphthalocyanin, Vitamin B12 und davon abgeleitete Derivate als Farbstoffe bevorzugt. Ebenfalls können weitere medizinisch zugelassene grünen, blaue, violette oder rote Farbstoffe verwendet werden. Besonders bevorzugt ist die Verwendung von Chloropyllin (E141), Brilliant grün BS (E142) und Brilliant blau (E133).

Die Erfindung wird durch nachstehende Beispiele erläutert, ohne jedoch die Erfindung dadurch zu beschränken.

### Beispiel 1

In einem 100 ml Erlenmeyerkolben werden 1,00 g Kupfer-Chlorophyllin (Farbstoff E141) mit 1,00 g Ölsäureethylester (Ph. Eu., Fluka) und 18,00 Methylmethacrylat (Fluka) unter Rühren innerhalb von 10 Minuten vermischt. Es entsteht eine tiefgrüne, klare Lösung. Diese Lösung wird dann mit 980 g Methylmethacrylat vermischt. Es entsteht eine sichtklare, intensiv grüne Lösung. Diese Lösung wird dann in 20 ml Ampullen überführt und die Ampullen werden zugeschmolzen. Die Ampullen werden als Monomerkomponente für einen PMMA-Knochenzement bereitgestellt.

### Beispiel 2

Eine grüne gefärbte Methylmethacrylat-Lösung, hergestellt entsprechend dem Beispiel 1, wurde mit Dibenzoylperoxid versetzt und bei 60 °C in Wasser unter intensivem Rühren zu Polymerperlen polymerisiert. Das entstandene Perlpolymerisat wird mit 30,0 Masseprozent Zirkoniumoxid und 1,0 Masseprozent Dibenzyolperoxid vermischt und als Pulverkomponente für einen PMMA-Knochenzement bereitgestellt.

## Patentansprüche

1. Polymethylmethacrylat-Knochenzement mit einer flüssigen Monomerkomponente und einer Feststoffkomponente, **dadurch gekennzeichnet**,
A dass in der Monomerflüssigkeit ein in Methacrylsäuremethylester nicht oder gering löslicher(s) Farbstoff/Farbstoffgemisch und ein synthetisch hergestellter, proteinfreier, hydrophober, niedermolekularer oder oligomerer Lösungsvermittler für den Farbstoff oder das Farbstoffgemisch gelöst sind,
B dass die Monomerflüssigkeit bei Raumtemperatur sichtklar ist, und
C dass ein in Methacrylsäuremethylester nicht oder gering löslicher(s) Farbstoff/Farb-stoffgemisch und ein synthetisch hergestellter, proteinfreier, hydrophober, niedermolekularer oder oligomerer Lösungsvermittler, homogen im Polymethacrylat oder Polymethylacrylat der Pulverkomponente gelöst ist.

2. Polymethylmethacrylat-Knochenzement nach Anspruch 1, wobei in der Monomerflüssigkeit bzw. im Polymethacrylat oder Polymethylacrylat der(das) Farbstoff/Farbstoffgemisch zu 0,001-1,00 Masseprozent, und der Lösungsvermittler zu 0,001-2,00 Masseprozent vorhanden sind.

3. Polymethylmethacrylat-Knochenzement nach Anspruch 1, wobei der Lösungsvermittler bei Raumtemperatur flüssig oder pastös ist.

4. Polymethylmethacrylat-Knochenzement mit einer flüssigen Monomerkomponente und einer Feststoffkomponente nach einem der Ansprüche 1-3, **dadurch gekennzeichnet, dass** der Farbstoff/Farbstoffgemisch und der synthetisch hergestellte, proteinfreie, hydrophobe, niedermolekulare oder oligomere Lösungsvermittler mit dem Farbstoff/Farbstoffgemisch in der Monomerflüssigkeit lösliche Addukte bildet.

5. Polymethylmethacrylat-Knochenzement mit einer flüssigen Monomerkomponente und einer Feststoffkomponente nach einem der Ansprüche 1-4, **dadurch gekennzeichnet, dass** Ölsäureester und/oder Elaidinsäureester und/oder Linolsäureester und/oder Linolensäureester der aliphatischen Alkohole mit 1 bis 22 Kohlenstoffatomen oder Oligomere dieser Ölsäureester als Löslichkeitsvermittler verwendet werden

6. Polymethylmethacrylat-Knochenzement mit einer flüssigen Monomerkomponente und einer Feststoffkomponente nach einem der Ansprüche 1-5, **dadurch gekennzeichnet, dass** Methacrylsäureester oder Acrylsäureester der aliphatischen Alkohole mit 4 bis 16 Kohlenstoffatomen oder Oligomere dieser Methacrylsäureester oder Acrylsäureester mit einer Molmasse kleiner 3000 g/mol als Löslichkeitsvermittler verwendet werden.

7. Polymethylmethacrylat-Knochenzement mit einer flüssigen Monomerkomponente und einer Feststoffkomponente nach einem der Ansprüche 1-6, **dadurch gekennzeichnet, dass** Glycerintrioleat, Glycerintrilinolat, Glycerintrilinolenat und Glycerintrielaidinat als Löslichkeitsvermittler verwendet werden.

8. Polymethylmethacrylat-Knochenzement mit einer flüssigen Monomerkomponete und einer Feststoffkomponente nach einem der Ansprüche 1-7, **dadurch gekennzeichnet, dass** der Löslichkeitsvermittler radikalisch polymerisierbare Doppelbindungen enthält.

9. Polymethylmethacrylat-Knochenzement mit einer flüssigen Monomerkomponete und einer Feststoffkomponente nach einem der Ansprüche 1-8, **dadurch gekennzeichnet, dass** als Farbstoffe Chlorophyllin, Indigo, Malachitgrün, Kristallviolett, Kupferphthalocyanin, Kobaltphthalocyanin, Vitamin B12 und davon abgeleitete Derivate als Farbstoffe verwendet werden.

## Claims

1. Polymethylmethacrylate bone cement having a liquid monomer component and a solid component, **characterised**
A **in that** a dye/dye mixture that is non-soluble or poorly soluble in methacrylic acid methylester and a synthetically produced, protein-free, hydrophobic, low-molecular or oligomeric solubiliser for the dye or the dye mixture are dissolved in said monomer liquid;
B **in that** the monomer liquid is clear to the view at room temperature; and
C **in that** a dye/dye mixture that is non-soluble or poorly soluble in methacrylic acid methylester and a synthetically produced, protein-free, hydrophobic, low-molecular or oligomeric solubiliser are dissolved homogeneously in the polymethacrylate or polymethylacrylate of the powder component.

2. Polymethylmethacrylate bone cement according to claim 1, whereby the amount of dye/dye mixture and the amount of solubiliser present in the monomer liquid or in the polymethacrylate or polymethylacrylate is 0.001 - 1.00 % by mass and 0.001 - 2.00 % by mass, respectively.

3. Polymethylmethacrylate bone cement according to claim 1, whereby the solubiliser is liquid or pasty at room temperature.

4. Polymethylmethacrylate bone cement having a liquid monomer component and a solid component according to any one of the claims 1-3, **characterised in that** the dye/dye mixture and the synthetically produced, protein-free, hydrophobic, low-molecular or oligomeric solubiliser form soluble adducts with the dye/dye mixture in the monomer liquid.

5. Polymethylmethacrylate bone cement having a liquid monomer component and a solid component according to any one of the claims 1-4, **characterised in that** oleic acid esters and/or elaidic acid esters and/or linoleic acid esters and/or lino-lenic acid esters of the aliphatic alcohols having 1 to 22 carbon atoms or oligomers of said oleic acid esters are present as solubiliser.

6. Polymethylmethacrylate bone cement having a liquid monomer component and a solid component according to any one of the claims 1-5, **characterised in that** methacrylic acid esters or acrylic acid esters of the aliphatic alcohols having 4 to 16 carbon atoms or oligomers of said methacrylic acid esters or acrylic acid esters having a molecular mass of less than 3,000 g/mol are used as solubiliser.

7. Polymethylmethacrylate bone cement having a liquid monomer component and a solid component according to any one of the claims 1-6, **characterised in that** glycerol trioleate, glycerol trilinolate, glycerol trilinolenate, and glycerol trielaidinate are used as solubiliser.

8. Polymethylmethacrylate bone cement having a liquid monomer component and a solid component according to any one of the claims 1-7, **characterised in that** the solubiliser contains double bonds that can be polymerised by radical polymerisation.

9. Polymethylmethacrylate bone cement having a liquid monomer component and a solid component according to any one of the claims 1-8, **characterised in that** chlorophyllin, indigo, malachite green, crystal violet, copper phthalocyanine, cobalt phthalocyanine, vitamin B12, and derivatives thereof are used as dyes.

## Revendications

1. Ciment osseux de méthacrylate de polyméthyle avec un composant monomère liquide et un composant solide, **caractérisé en ce**
A **qu'**un colorant/mélange de colorants non soluble ou faiblement soluble dans l'ester méthylique d'acide méthacrylique et un agent de solubilisation fabriqué par voie synthétique, exempt de protéine, hydrophobe, à faible poids moléculaire ou oligomère pour le colorant ou le mélange de colorants sont dissous dans le liquide monomère,
B **que** le liquide monomère est clair à température ambiante, et
C **qu'**un colorant/mélange de colorants non soluble ou faiblement soluble dans l'ester méthylique d'acide méthacrylique et un agent de solubilisation fabriqué par voie synthétique, exempt de protéine, hydrophobe, à faible poids moléculaire ou oligomère sont dissous de manière homogène dans le polyméthacrylate ou le polyméthylacrylate du composant pulvérulent.

2. Ciment osseux de méthacrylate de polyméthyle selon la revendication 1, dans lequel le colorant/mélange de colorants est présent dans le liquide monomère ou dans le polyméthacrylate ou le polyméthylacrylate à un pourcentage massique de 0,001 à 1,00 et l'agent de solubilisation à un pourcentage massique de 0,001 à 2,00.

3. Ciment osseux de méthacrylate de polyméthyle selon la revendication 1, dans lequel l'agent de solubilisation est liquide ou pâteux à température ambiante.

4. Ciment osseux de méthacrylate de polyméthyle avec un composant monomère liquide et un composant solide selon l'une quelconque des revendications 1 à 3, **caractérisé en ce que** le colorant/mélange de colorants et l'agent de solubilisation fabriqué par voie synthétique, exempt de protéine, hydrophobe, à faible poids moléculaire ou oligomère forment avec le colorant/mélange de colorants des produits d'addition solubles dans le liquide monomère.

5. Ciment osseux de méthacrylate de polyméthyle avec un composant monomère liquide et un composant solide selon l'une quelconque des revendications 1 à 4, **caractérisé en ce que** des esters d'acide oléique et/ou des esters d'acide élaïdique et/ou des esters d'acide linoléique et/ou des esters d'acide linolénique des alcools aliphatiques avec 1 à 22 atomes de carbone ou des oligomères de ces esters d'acide oléique sont utilisés en tant qu'agent de solubilisation.

6. Ciment osseux de méthacrylate de polyméthyle avec un composant monomère liquide et un composant solide selon l'une quelconque des revendications 1 à 5, **caractérisé en ce que** des esters d'acide méthacrylique ou des esters d'acide acrylique des alcools aliphatiques avec 4 à 16 atomes de carbone ou des oligomères de ces esters d'acide méthacrylique ou esters d'acide acrylique avec une masse molaire inférieure à 3000 g/mole sont utilisés en tant qu'agent de solubilisation.

7. Ciment osseux de méthacrylate de polyméthyle avec un composant monomère liquide et un composant solide selon l'une quelconque des revendications 1 à 6, **caractérisé en ce que** le trioléate de glycérine, le trilinolate de glycérine, le trilinolénate de glycérine et l'élaïdinate de glycérine sont utilisés en tant qu'agent de solubilisation.

8. Ciment osseux de méthacrylate de polyméthyle avec un composant monomère liquide et un composant solide selon l'une quelconque des revendications 1 à 7, **caractérisé en ce que** l'agent de solubilisation contient des doubles liaisons polymérisables par voie radicalaire.

9. Ciment osseux de méthacrylate de polyméthyle avec un composant monomère liquide et un composant solide selon l'une quelconque des revendications 1 à 8, **caractérisé en ce que** de la chlorophylline, de l'indigo, du vert de malachite, du violet cristallin, de la phtalocyanine de cuivre, de la phtalocyanine de cobalt, de la vitamine B12 et des dérivés qui en sont dérivés sont utilisés en tant que colorants.
